(19) Europäisches Patentamt
European Patent Office
Office européen des brevets

(11) Veröffentlichungsnummer: **0 242 586**
**B1**

(12) # EUROPÄISCHE PATENTSCHRIFT

(45) Veröffentlichungstag der Patentschrift: **27.12.90**

(51) Int. Cl.⁵: **A61K 7/48**, A61K 7/50, C11D 1/94

(21) Anmeldenummer: **87103910.3**

(22) Anmeldetag: **17.03.87**

(54) **Mittel mit reinigender und hautpflegender Eigenschaft.**

(30) Priorität: **27.03.86 DE 3610395**

(43) Veröffentlichungstag der Anmeldung:
**28.10.87 Patentblatt 87/44**

(45) Bekanntmachung des Hinweises auf die Patenterteilung:
**27.12.90 Patentblatt 90/52**

(84) Benannte Vertragsstaaten:
**AT BE CH DE ES FR GB GR IT LI LU NL SE**

(56) Entgegenhaltungen:
**EP-A- 0 117 135**
**EP-A- 0 122 324**
**US-A- 3 313 734**
**US-A- 4 110 263**
**US-A- 4 157 388**
**US-A- 4 166 845**

(73) Patentinhaber: **Wella Aktiengesellschaft, Berliner Allee 65, D-6100 Darmstadt(DE)**

(72) Erfinder: **Greiche, Youssef, Dr., Soderstrasse 36, D-6100 Darmstadt(DE)**
Erfinder: **Schwarz, Horst, Fliederweg 22 D, D-6104 Seeheim(DE)**
Erfinder: **Hölzel, Hans, Jahnstrasse 9, D-6101 Fränkisch-Crumbach(DE)**

EP 0 242 586 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

## Beschreibung

Die Erfindung betrifft ein Mittel mit reinigender und hautpflegender Eigenschaft auf der Basis einer Kombination von Tensiden und eines kationischen Polymers, welche vorzugsweise als Schaum- oder Duschbad verwendet werden kann.

Schaum- und Duschbadzusammensetzungen enthalten als reinigende Wirkstoffe üblicherweise anionische Tenside, beispielsweise Alkalisalze von Alkyl- oder Alkylethersulfaten, Di-Natriumsalze von Sulfobernsteinsäurehalbestern oder Eiweiß/Fettsäure-Kondensationsprodukte.

Weiterhin können in höherwertigen Schaum- und Duschbadzusammensetzungen nichtionogene und amphotere Tenside enthalten sein. Als nichtionogene Tenside sind beispielsweise Fettsäurealkylolamide, Aminoxide, Polyglycerylether, gesättigte oder ungesättigte Fettalkohole und Alkylphenole zu nennen, während zu den amphoteren Tensiden beispielsweise Alkylamidobetaine, Sulfobetaine, Alkylimidazolinium-betaine und N-Alkyl-beta-aminopropionsäure zählen.

Derartige Schaum- und Duschbadzusammensetzungen sind beispielsweise in der Literatur H Janistyn, "Handbuch der Kosmetika und Riechstoffe", (1973) 3. Band, Seiten 442 - 453 und Seifen-Öle-Fette-Wachse 94, (1968) Seite 475 ff. beschrieben.

Die Verwendung der oben genannten Tenside in Schaum- oder Duschbadzusammensetzungen, alleine oder als Gemisch, kann jedoch beim Anwender zu einem Spannungsgefühl und Austrocknen der Haut führen. Dieser Nachteil soll durch den Zusatz von pflegend wirkenden Bestandteilen, wie sie beispielsweise in der Literatur H. Janistyn, "Handbuch der Kosmetika und Wirkstoffe", 3. Band, (1973) Seiten 445 ff., der Firmenschrift der Firma Henkel, "Kosmetik Modell-Rezepturen", Ausgabe 1979, und in der DE-OS 3 302 456 empfohlen sind, behoben werden. Als pflegende Bestandteile werden unter anderem ethoxylierte und propoxylierte gesättigte Fettalkohole, Fettsäureester, Fettsäureglyceride, Fettsäureisopropylester, Celluloseether, quarternäre Celluloseether, Chitinderivate und quarternäre Chitinderivate genannt.

Allerdings ist der subjektive Pflegeeindruck auf der Haut nach der Anwendung von Schaum- und Duschbadzusammensetzungen mit den vorstehend genannten pflegenden Bestandteilen gering und wird daher vom Anwender kaum wahrgenommen.

Stark pflegend wirkende Zusammensetzungen, welche ein deutliches, angenehmes und gepflegtes Hautgefühl vermitteln, sind Ölbäder und Öl-Schaumbäder. Die reinen Ölbäder besitzen jedoch keine reinigende Wirkung und die Öl-Schaumbäder sind aufgrund ihrer lipophilen Zusammensetzungen nur auf ganz spezielle wasserarme Tensidlösungen und Ölkomponenten beschränkt.

Bei derartigen Öl-Schaumbädern, welche beispielsweise aus der DE-OS 2 943 202 und der EP-PS 26 073 bekannt sind, handelt es sich im allgemeinen um einphasige Zusammensetzungen in denen der Wassergehalt auf maximal 15 Gewichtsprozent beschränkt ist, um die Klarlöslichkeit der ölkomponenten in den Tensiden zu gewährleisten.

Aufgabe der Erfindung war es daher, ein Mittel mit reinigender und hautpflegender Eigenschaft zur Verfügung zu stellen, welches den Vorteil von reinen ölbädern oder öl-Schaumbädern, nämlich eine starke hautpflegende Wirkung, mit dem Vorteil reiner Schaum- und Duschbäder, nämlich ein ausgezeichnetes Schaumverhalten, vereint, ohne spezielle wasserarme Tensidlösungen und eine Ölkomponente verwenden zu müssen.

Es wurde nun gefunden, daß ein Mittel mit reinigender und hautpflegender Eigenschaft auf der Basis einer Kombination von Tensiden und kationischen Polymeren, gekennzeichnet durch einen Gehalt an

A) 12,0 - 24,0 Gewichtsprozent eines Alkylamidobetains der allgemeinen Formel (I)

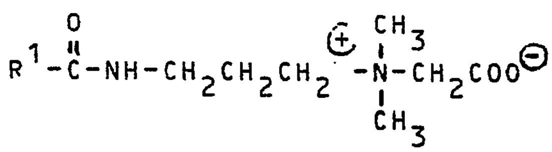

(I),

worin $R^1$ einen Alkylrest mit 7 bis 17 Kohlenstoffatomen darstellt,

B) 1,0 - 3,0 Gewichtsprozent eines amphoteren Carboxyglycinats der allgemeinen Formel (II)

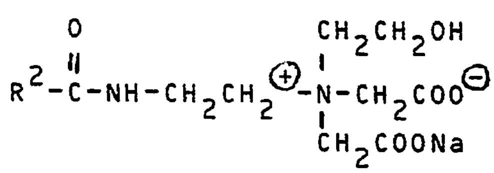

(II),

worin $R^2$ einen Alkylrest mit 7 bis 17 Kohlenstoffatomen darstellt,

C) 2,0 - 6,0 Gewichtsprozent eines Alkylsulfatsalzes der allgemeinen Formel (III)

$$R^3-OSO_3^{\ominus}M^{\oplus} \qquad (III),$$

worin R³ einen Alkylrest mit 8 bis 18 Kohlenstoffatomen darstellt, und M die Bedeutung Natrium, Kalium oder Ammonium hat oder aber einen Mono-, Di- oder Trialkanolammoniumrest darstellt,

D) 1,0 - 2,0 Gewichtsprozent eines polyquarternären Harnstoffderivates der allgemeinen Formel (IV)

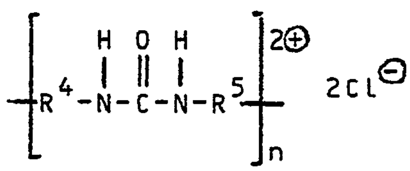

(IV),

worin n eine ganze Zahl von 1 bis 10, vorzugsweise 6, darstellt,

$R^4$ =

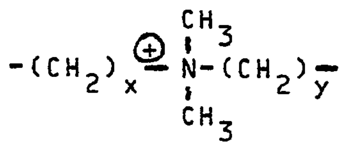

ist, und

$R^5$ =

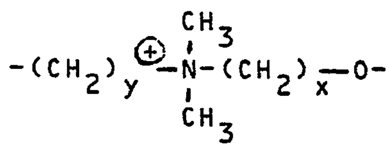

bedeutet, worin x eine ganze Zahl von 2 bis 4, vorzugsweise 2, ist und y eine ganze Zahl von 1 bis 5, vorzugsweise 3, darstellt, und

E) 65,0 - 84,0 Gewichtsprozent Wasser,

der gestellten Aufgabe in hervorragendem Maße gerecht wird.

Als Alkylamidobetain der Komponente A) kommt insbesondere das 1-Kokosamido-3-(dimethyl-carboxymethyl) ammonio-propan, welches beispielsweise in Form einer 30-prozentigen wäßrigen Lösung unter der Bezeichnung "Tego® Betain L 7" von der Firma Th. Goldschmidt AG, D-4300 Essen, vertrieben wird, in Betracht.

Bevorzugt ist die Komponente B) des vorliegenden Mittels das 1-Kokosamido-2-(dicarboxymethyl-2'-hydroxyethyl)-ammonio-ethan, welches zum Beispiel in Form einer 50-prozentigen wäßrigen Lösung unter der Bezeichnung "Miranol® C2M" von der Firma Miranol Chemical Company Inc , New Yersey, USA vertrieben wird.

Das bevorzugte Alkylsulfatsalz der Komponente C) ist das Natriumlaurylsulfat.

Als geeignetes polyquaternäres Harnstoffderivat für die Komponente D) ist besonders das Poly N-[3-(dimethylammonio)propyl]- N'-[3-(ethylenoxyethylen-dimethylammonio)propyl]harnstoff-dichlorid zu nennen, welches beispielsweise in Form der 64-prozentigen wäßrigen Lösung unter der Bezeichnung "Mirapol® A - 15" ebenfalls von der Firma Miranol vertrieben wird.

Das erfindungsgemäße Mittel eignet sich insbesondere zur Verwendung als Schaum- oder Duschbad und weist neben einem hervorragenden Schaumvermögen eine sehr gute pflegende Wirkung auf die Haut auf. Weiterhin verursacht das Mttel nach seiner Anwendung weder ein Spannungsgefühl der Haut noch trocknet es die Haut aus.

Wird das hier beschriebene Mittel als Schaumbad eingesetzt, so beträgt das Verhältnis Schaumbad/Badewasser etwa 20 ml/150 l, während beim Einsatz als Duschbad etwa 5 ml des Mittels benötigt werden.

Der pH-Wert des erfindungsgemäßen Mittels beträgt etwa 4,0 bis 7,0, vorzugsweise 5,0 bis 6,0. Die Einstellung des Mittels auf den gewünschten pH-Wert erfolgt beispielsweise mit Zitronensäure, Phosphorsäure, Salzsäure oder Natriumhydroxid.

Selbstverständlich kann das erfindungsgemäße Mittel neben den genannten Bestandteilen auch übliche kosmetische Zusätze, beispielsweise Parfümöle in einer Menge von etwa 0,5 bis 5,0 Gewichtsprozent, Trübungsmittel, wie z. B. Ethylenglykoldistearat, in einer Menge von etwa 0,5 bis 5,0 Gewichtsprozent, Perlglanzmittel, wie z. B. ein Gemisch aus Fettsäuremonoalkylolamid/Ethylenglykoldistearat, in einer Menge von etwa 1,0 bis 10,0 Gewichtsprozent, bakterizide oder fungizide Wirkstoffe, wie z. B. 2,4,4-Trichlor-2-hydroxy-diphenylether oder Methylchlorisothiazolinon, in einer Menge von etwa 0,01 bis 1,0 Gewichtsprozent, Verdickungsmittel, wie beispielsweise Kokosfettsäurediethanolamid, in einer Menge von etwa 0,5 bis 3,0 Gewichtsprozent, Verdünnungsmittel, wie z. B. 1,2-Propylenglykol oder ethoxyliertes Sorbitanmonolaurat, in einer Menge von etwa 0,5 bis 5,0 Gewichtsprozent, Puffersubstanzen, wie beispielsweise Natriumcitrat oder Natriumphosphat, in einer Menge von etwa 0,1 bis 1,0 Gewichtsprozent, Lösungsvermittler, wie z. B. ethoxyliertes, gegebenenfalls hydriertes Rizinusöl, in einer Menge von etwa 0,1 bis 1,0 Gewichtsprozent sowie Anfärbestoffe, wie z. B. Fluorescein-Natriumsalz, in einer Menge von etwa 0,1 bis 1,0 Gewichtsprozent, enthalten.

Weitere übliche, für derartige Mittel bekannte Bestandteile, welche in dem neuen Mittel enthalten sein können, sind beispielsweise bei H. Janistyn, "Handbuch der Kosmetika und Riechstoffe", 3. Band, (1973), Seiten 442 - 462, K. Schrader, "Grundlagen und Rezepturen der Kosmetika", (1979) Seiten 375 - 401 sowie G. A. Nowak, "Die kosmetischen Präparate", (1975) Seiten 274 ff., beschrieben.

Zum Nachweis der überlegenen, reinigenden und hautpflegenden Eigenschaften des erfindungsgemäßen Mittels, welche nur durch den Einsatz der Komponenten A) bis E) in dem vorstehend genannten Gewichtsverhältnis erzielbar sind, wurden Vergleichsversuche durchgeführt, die im Folgenden beschrieben werden.

Beispiel 1

Der subjektive Pflegeeindruck auf der Haut nach der Anwendung einer erfindungsgemäßen Schaumbadzusammensetzung (X) im Vergleich zu einer dem Stand der Technik entsprechenden Schaumbadzusammensetzung (Y) wurde von 182 Testpersonen im Alter von 20 - 45 Jahren beurteilt. Die Testzusammensetzungen waren neutral abgefüllt und wurden durchschnittlich 3-mal bei einer mittleren Badedauer von 20 Minuten benutzt.

Das erfindungsgemäße Mittel (X) hatte folgende Zusammensetzung:

| | | |
|---|---|---|
| (a) | 58,0 g | 1-Kokosamido-3-(dimethyl-carboxymethyl) ammonio-propan, 30-prozentige wäßrige Lösung |
| (b) | 5,8 g | 1-Kokosamido-2-(dicarboxymethyl-2'-hydroxy-ethyl)ammonio-ethan, 50-prozentige wäßrige Lösung |
| (c) | 4,5 g | Natriumlaurylsulfat, fest, 90,5-prozentig |
| (d) | 2,2 g | Poly N-[3-(dimethylammonio)propyl]-N'-[3-(ethylenoxyethylen-dimethylammonio)propyl] harnstoff-dichlorid, 64-prozentige wäßrige Lösung |
| | 2,0 g | Trübungsmittel |
| | 1,0 g | Parfümöl, Farbe |
| (e) | 26,5 g | Wasser |
| | 100,0 g | |

Ein Schaumbad nach dem Stand der Technik (Y), dessen Bestandteile in ähnlicher Zusammensetzung, beispielsweise aus der Firmenschrift der Firma Henkel "Kosmetik Modell-Rezepturen" (1979), Seiten 181, 182, und der österreichischen Patentanmeldung 5283/80 bekannt sind, war wie folgt aufgebaut:

| | |
|---|---|
| 57,0 g | Natriumlaurylethersulfat, 28-prozentige wäßrige Lösung |
| 3,5 g | Mit 30 Mol Ethylenoxid oxethylierter Monokokosfettsäureglycerylester |
| 23,0 g | Eiweiß/Fettsäurekondensationsprodukt, 30-prozentige wäßrige Lösung |
| 0,5 g | Trübungsmittel |
| 2,5 g | Parfüm, Farbe |
| 0,1 g | Konservierungsmittel |
| 13,4 g | Wasser |
| 100,0 g | |

Während das Auflösungs- und Schaumvermögen der Schaumbadzusammensetzungen (X) und (Y) von den Testpersonen als etwa gleich gut beurteilt wurde, schnitt die erfindungsgemäße Schaumbadzusammensetzung (X) sowohl bezüglich der Weichheit und Geschmeidigkeit der Haut nach der Benutzung und bezüglich der pflegenden Eigenschaften als auch im Gesamturteil deutlich besser ab, als die Vergleichszusammensetzung (Y). Dies wird durch die in den folgenden Tabellen angeführten Testergebnisse eindeutig belegt.

**Tabelle 1**

Beurteilung der Weichheit und Geschmeidigkeit der Haut nach der Anwendung:

| Schaumbad-zusammen-setzung | sehr gut | gut | mittel-mäßig | schlecht | sehr schlecht |
|---|---|---|---|---|---|
| (X) | 53 | 52 | 56 | 18 | 3 |
| (Y) | 18 | 49 | 69 | 36 | 10 |

Tabelle 2

Beurteilung der hautpflegenden Eigenschaften der Schaumbadzusammensetzungen:

| Schaumbadzusammensetzung | sehr gut | gut | mittelmäßig | schlecht | sehr schlecht |
|---|---|---|---|---|---|
| (X) | 60 | 91 | 26 | 5 | - |
| (Y) | 30 | 83 | 48 | 18 | 3 |

Tabelle 3

Gesamturteil:

| Schaumbadzusammensetzung | sehr gut | gut | mittelmäßig | schlecht | sehr schlecht |
|---|---|---|---|---|---|
| (X) | 54 | 87 | 30 | 11 | - |
| (Y) | 27 | 75 | 51 | 27 | 2 |

Eine weitere Bestätigung für die hervorragende pflegende Wirkung der erfindungsgemäßen Schaumbadzusammensetzung (X) zeigt die Häufigkeit der Verwendung einer Körperlotion nach dem Baden. Während nach der Verwendung der Schaumbadzusammensetzung nach dem Stand der Technik (Y) 99 Testpersonen ein Körperlotion benutzten, war dies bei der Verwendung der neuen Schaumbadzusammensetzung (X) lediglich bei 72 Testpersonen der Fall.

Ein weiterer Vergleichsversuch wurde durchgeführt, indem das erfindungsgemäße Schaumbad (X) in seinen kosmetischen Eigenschaften mit einem nachfolgend angegebenen Produkt ähnlicher Zusammensetzung (Z), jedoch auf der Basis eines Alkylethersulfattensids nach dem Stand der Technik, verglichen wurde.

| | | |
|---|---|---|
| (a) | 15,0 g | Kokosfettsäureamidopropyl-betain, 30-prozentige wäßrige Lösung |
| (b) | 10,5 g | 1-Kokosamido-2-(dicarboxymethyl-2'-hydroxyethyl)ammonio-ethan, 50-prozentige wäßrige Lösung |
| | 21,0 g | Natriumlaurylethersulfat, 27-prozentige wäßrige Lösung |
| (d) | 2,1 g | Poly N-[3-(dimethylammonio)propyl]-N'-[3-(ethylenoxyethylen-dimethylammonio)propyl]-harnstoff-dichlorid, 64-prozentige wäßrige Lösung |
| | 51,4 g | Wasser |
| | 100,0 g | |

Ergebnis: Von allen Testpersonen wurde die mangelhafte Schaumbildung der Zusammensetzung (Z) bemängelt. Die hautpflegenden Eigenschaften der erfindungsgemäßen Zusammensetzung (X) wurden deutlich besser beurteilt als diejenigen der Zusammensetzung (Z).

Während in dem vorliegenden Mittel (X) als waschaktive Substanz ein Alkylsulfatsalz enthalten ist, wird in den Vergleichszusammensetzungen (Y) und (Z) ein Alkylethersulfatsalz verwendet. Es ist bekannt, daß sowohl Alkylsulfatsalze als auch Alkylethersulfatsalze sehr gute Wascheigenschaften aufweisen. Im Gegensatz zu den Alkylsulfatsalzen sind die Alkylethersulfatsalze (siehe zum Beispiel K. Schrader, Seifen-Öle-Fette-Wachse 111, (1985) 41) weitaus besser hautverträglich und rauhen die Haut weniger auf. Die eindeutige Überlegenheit des erfindungsgemäßen Mittels mit einem Gehalt an Alkylsulfatsalz bezüglich der hautpflegenden Eigenschaften ist somit überraschend.

**Beispiel 2** Schaum- und Duschbadzusammensetzung

| | | |
|---|---|---|
| (a) | 55,0 g | 1-Kokosamido-3-(dimethyl-carboxymethyl) ammonio-propan, 30-prozentige wäßrige Lösung |
| (b) | 6,0 g | 1-Kokosamido-2-(dicarboxymethyl-2'-hydroxy-ethyl)ammonio-ethan, 50-prozentige wäßrige Lösung |
| (c) | 4,5 g | Natriumlaurylsulfat, fest, 90,5-prozentig |
| (d) | 2,2 g | Poly N-[3-(dimethylammonio)propyl] N'-[3-(ethylenoxyethylen-dimethylammonio)propyl] harnstoff-dichlorid, 64-prozentige wäßrige Lösung |
| (e) | 32,3 g | Wasser |
| | 100,0 g | |

Zur Herstellung der obigen Schaum- und Duschbadzusammensetzung wird zunächst Komponente (c) im Wasser gelöst. Diese Lösung wird anschließend in die homogene Mischung der Komponenten (a), (b) und (d) eingerührt. Alle Löse- und Mischvorgänge erfolgen bei Raumtemperatur.

Die auf diese Weise erhaltene Schaum- und Duschbadzusammensetzung besitzt ein sehr gutes Schaumvermögen und hinterläßt nach der Anwendung ein hervorragendes pflegendes Hautgefühl.

Alle Prozentangaben dieser Anmeldung stellen, soweit nicht anders angegeben, Gewichtsprozent dar.

**Patentansprüche**

1. Mittel mit reinigender und hautpflegender Eigenschaft auf der Basis einer Kombination von Tensiden und kationischen Polymeren, gekennzeichnet durch einen Gehalt an

(A) 12,0 - 24,0 Gewichtsprozent eines Alkylamidobetains der allgemeinen Formel (I)

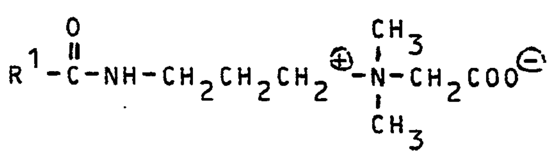 (I),

worin $R^1$ einen Alkylrest mit 7 bis 17 Kohlenstoffatomen darstellt,

(B) 1,0 - 3,0 Gewichtsprozent eines amphoteren Carboxyglycinats der allgemeinen Formel (II)

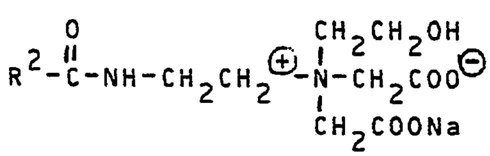 (II),

worin $R^2$ einen Alkylrest mit 7 bis 17 Kohlenstoffatomen darstellt,
(C) 2,0 - 6,0 Gewichtsprozent eines Alkylsulfatsalzes der allgemeinen Formel (III)

$$R^3-OSO_3^{\ominus}M^{\oplus} \quad (III),$$

worin $R^3$ einen Alkylrest mit 8 bis 18 Kohlenstoffatomen darstellt und M die Bedeutung Natrium, Kalium oder Ammonium hat oder aber einen Mono-, Di- oder Trialkanolammoniumrest darstellt,
(D) 1,0 - 2,0 Gewichtsprozent eines polyquarternären Harnstoffderivates der allgemeinen Formel (IV)

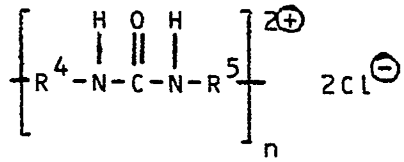
(IV),

worin n eine ganze Zahl von 1 bis 10, vorzugsweise 6, darstellt,

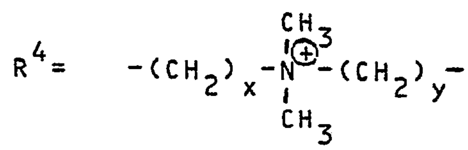

ist, und

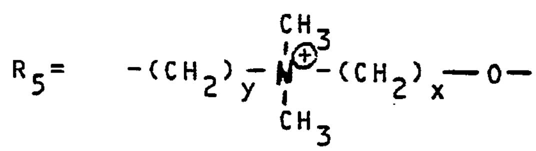

bedeutet, worin x eine ganze Zahl von 2 bis 4, vorzugsweise 2, ist und y eine ganze Zahl von 1 bis 5, vorzugsweise 3, darstellt, und
(E) 65,0 - 84,0 Gewichtsprozent Wasser.

**Claims**

1. Cleansing and skin-conditioning composition based on a combination of tensides and cationic polymers, characterised by a content of
(A) 12,0 to 24,0% by weight of an alkyl amido betaine of the general formula (I)

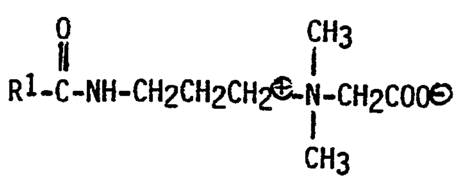
(I),

in which $R^1$ is an alkyl radical containing 7 to 17 carbon atoms,
(B) 1,0 to 3,0% by weight of an amphoteric carboxy glycinate of the general formula (II)

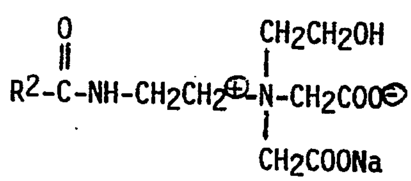
(II),

in which $R^2$ is an alkyl radical containing 7 to 17 carbon atoms,
(C) 2,0 to 6,0% by weight of an alkyl sulphate salt of the general formula (III),

$R^3-OSO_3^{\ominus}M^{\oplus}$ (III),

in which $R^3$ is an alkyl radical containing 8 to 18 carbon atoms and M denotes sodium, potassium or ammonium or represents a mono-, di- or trialkanol ammonium radical,
(D) 1,0 to 2,0% by weight of a polyquaternary urea derivative of the general formula (IV)

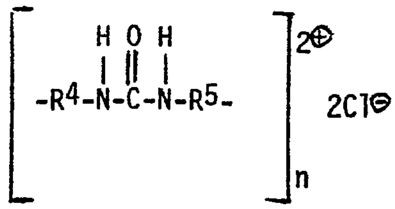

(IV),

in which n is a whole number from 1 to 10, preferably 6, $R^4$ represents

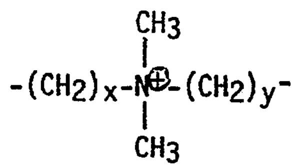

and $R^5$ represents

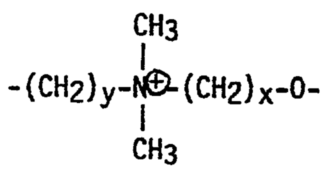

,

in which x is a whole number from 2 to 4, preferably 2, while y is a whole number form 1 to 5, preferably 3, and
(E) 65 to 84% by weight water.

**Revendications**

1. Composition pour le nettoyage et le soin de la peau à base d'une combinaison d'agents tensio-actifs et de polymères cationiques, caractérisé en ce qu'il renferme:
(A) 12,0 à 24,0% en poids d'une alkylamidobétaine répondant à la formule générale (I)

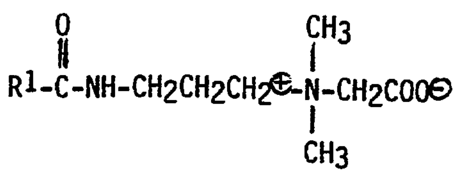

(I),

dans laquelle $R^1$ représente un reste alkylique comportant 7 à 17 atomes de carbone,
(B) 1,0 à 3,0% en poids d'un carboxyglycinate amphotère répondant à la formule générale (II)

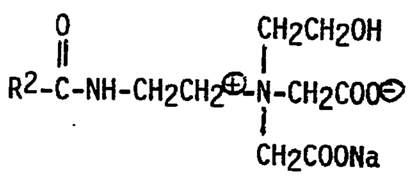

(II),

dans laquelle $R^2$ représente un reste alkylique comportant 7 à 17 atomes de carbone,
(C) Comme sel, 2,0 à 6,0% en poids d'un alkylsulfate, répondant à la formule générale (III)

$$R^3-OSO_3^{\ominus}M^{\oplus} \quad \text{(III)},$$

dans laquelle $R^3$ représente un reste alkylique comportant, 8 à 18 atomes de carbone et M désigne le sodium, le potassium, ou un groupe ammonium ou encore représente un reste mono-, di- ou tri-alcanol-ammonium,

(D) 1,0 à 2,0% en poids d'un dérivé polyquaternaire de l'urée, répondant à la formule générale (IV)

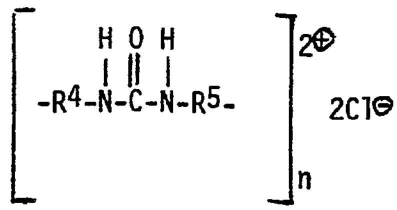

(IV),

dans laquelle n représente un nombre entier de 1 à 10, de préférence égal à 6, $R^4$ répresente

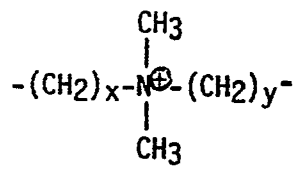

et $R^5$ répresente

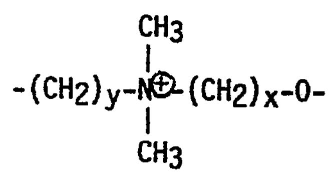

,

ou x est un nombre entier de 2 à 4, de préférence égal à 2, et y est un nombre entier de 1 à 5, de préférence égal à 3, et

(E) 65 à 84% en poids d'eau.